(19) Europäisches Patentamt
European Patent Office
Office européen des brevets

(11) EP 2 090 300 A1

(12) EUROPEAN PATENT APPLICATION
published in accordance with Art. 153(4) EPC

(43) Date of publication:
**19.08.2009 Bulletin 2009/34**

(21) Application number: **07832746.7**

(22) Date of filing: **29.11.2007**

(51) Int Cl.:
***A61K 9/22*** (2006.01) ***A61K 31/506*** (2006.01)
***A61K 47/10*** (2006.01) ***A61K 47/38*** (2006.01)
***A61P 7/02*** (2006.01)

(86) International application number:
**PCT/JP2007/073031**

(87) International publication number:
**WO 2008/066102 (05.06.2008 Gazette 2008/23)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **30.11.2006 JP 2006323815**

(71) Applicant: **Takeda Pharmaceutical Company Limited**
**Osaka- shi, Osaka 541-0045 (JP)**

(72) Inventors:
- **IINUMA, Satoshi**
  **deceased (JP)**
- **YOSHINARI, Tomohiro**
  **Osaka-shi**
  **Osaka 532-8686 (JP)**
- **TOTTORI, Tsuneaki**
  **Osaka-shi**
  **Osaka 532-8686 (JP)**

(74) Representative: **Jones, Nicholas Andrew et al**
**Withers & Rogers LLP**
**Goldings House**
**2 Hays Lane**
**London**
**SE1 2HW (GB)**

# (54) SUSTAINED RELEASE PREPARATION

(57) Disclosed is a sustained-release preparation which is prepared by shaping a granule comprising a blood coagulation factor Xa inhibitor and a mixture of at least two hydrophilic polymers. Also disclosed is a pharmaceutical composition comprising a combination of the sustained-release preparation and an immediate release preparation comprising a blood coagulation factor Xa inhibitor. It becomes possible to provide a controlled release preparation comprising a blood coagulation factor Xa inhibitor for the prevention or treatment of thrombosis, which can control the activity of blood coagulation factor Xa for a long term and is excellent in convenience and compliance. It is also becomes possible to provide a method for producing the controlled release preparation.

EP 2 090 300 A1

## Description

Technical Field

The present invention relates to a sustained-release preparation comprising a blood coagulation factor Xa inhibitor useful for the prevention or treatment of thrombosis.

Background Art

According to the data surveyed about the rate to the total number of death (Medicine Ranking, 2002 edition, Mikusu Co.), the second and third leading causes of death are a cardiac disease and cerebrovascular disease in Japan, and the disease pathogenesis thereof is a thrombus. In a cerebral infarction having a high potential to cause an aftereffect, there is a problem in QOL (quality of life) of the patient and family, and a more excellent antithrombotic drug is desired. Although an antiplatelet drug and the like are currently the mainstream of the antithrombotic drug, research and development of inhibitors targeting a blood coagulation factor Xa (hereinafter, occasionally abbreviated as FXa) located upstream of the blood coagulation cascade are attracting attention. The FXa is a factor that activates thrombin in blood, and since the thrombin forms thrombi by converting fibrinogen to fibrin, the FXa is a target protein of anticoagulants. Since the FXa inhibitor is a FXa inhibiting agent exerting a selective thrombogenesis inhibitory action in the patient with venous thrombosis (patient having three main factors, i.e., injury of venous inner layer, enhancement of blood coagulation tendency and decrease of blood flow rate that are known as three main features of Virchow), the sustained-release preparation of the present invention is useful as therapeutic agent for thrombosis without bleeding risk due to suppression of thrombogenesis without activating platelets.

In a general anticoagulant therapy, a low molecular weight heparin is injected subcutaneously, and then an oral drug warfarin is administered orally. For the treatment of deep vein thrombosis, in the event that it is developed once, after a low molecular weight heparin is injected subcutaneously, an administration of oral drug warfarin may be required for two months. When a pulmonary embolism is developed after deep vein thrombosis, the administration may be required for six months because the high risk condition of deep vein thrombosis is to be continued. Further, when deep vein thrombosis is developed two or more times, continuous administration of warfarin may be required for an indefinite period. However, the administration of warfarin increases the risk of bleeding regardless of internal bleeding or external bleeding, thus in order to keep the risk as low as possible, it is necessary to measure the coagulation time by conducting a blood test periodically and adjust the dose of warfarin based on the result.

In addition, when a FXa inhibitor is used for a prevention or treatment of thrombosis, multiple administration of the FXa inhibitor is usually needed.

Disclosure of Invention

In view of the current situation that it is not necessarily desirable for living body to simply strongly inhibit FXa in living body, inhibition of FXa activity in living body needs to be done appropriately to prevent or treat thrombosis effectively. That is, in consideration of the balance between extent and time to suppress thrombogenesis and prolongation of bleeding time as a side effect, it was needed to develop a preparation that appropriately inhibits FXa activity and is excellent in convenience or compliance.

As a result of research on a preparation that inhibits FXa activity appropriately and can be administered once a day, the present inventors found that a sustained-release preparation exerting the desired effects can be obtained by combining a FXa inhibitor and a hydrophilic polymer, and further studied to complete the present invention.

That is, the present invention relates to:

(1) A sustained-release preparation comprising a blood coagulation factor Xa inhibitor and a hydrophilic polymer,
(2) The sustained-release preparation according to the above-mentioned (1), wherein the blood coagulation factor Xa inhibitor is 1-(1-{(2S)-3-[(6-chloronaphthalen-2-yl)sulfonyl]-2-hydroxypropanoyl}piperidin-4-yl)tetrahydropyrimidin-2(1H)-one,
(3) The sustained-release preparation according to the above-mentioned (1), wherein a content of the hydrophilic polymer in the preparation is 5 to 90% by weight,
(4) A pharmaceutical composition which comprises a combination of preparations containing two or more blood coagulation factor Xa inhibitors, which have a different release rate of the blood coagulation factor Xa inhibitor,
(5) The pharmaceutical composition according to the above-mentioned (4), wherein the blood coagulation factor Xa inhibitor is 1-(1-{(2S)-3-[(6-chloronaphthalen-2-yl)sulfonyl]-2-hydroxypropanoyl}piperidin-4-yl)tetrahydropyrimidin-2(1H)-one,
(6) The pharmaceutical composition according to the above-mentioned (4), which comprises a combination of a

sustained-release preparation containing a blood coagulation factor Xa inhibitor and a quick-release preparation containing a blood coagulation factor Xa inhibitor,

(7) A controlled-release preparation containing a blood coagulation factor Xa inhibitor, which can maintain a plasma level of the blood coagulation factor Xa inhibitor at 1.25 nmol/mL or less over one hour to 24 hours after the administration and can reduce the blood coagulation factor Xa activity by 10% or more than usual over one hour to 24 hours after the administration,

(8) The preparation according to the above-mentioned (7), wherein the blood coagulation factor Xa inhibitor is 1-(1-{(2S)-3-[(6-chloronaphthalen-2-yl)sulfonyl]-2-hydroxypropanoyl}piperidin-4-yl)tetrahydropyrimidin-2(1H)-one,

(9) The preparation according to the above-mentioned (1) or (7), which is for prevention or treatment of thrombosis,

(10) The preparation according to the above-mentioned (1) or (7), which is a secondary prophylactic agent of acute coronary syndrome,

(11) A process for production of a sustained-release preparation, comprising:

a) a step for granulating a mixture comprising an active ingredient and mannitol with spraying an aqueous solution of hydroxypropyl cellulose, and

b) a step for formulating a mixture comprising the granulated material obtained in step a) and 2 or more hydrophilic polymers to obtain a formulated substance,

(12) The process for production according to the above-mentioned (11), wherein the 2 or more hydrophilic polymers are hydroxypropyl cellulose and hydroxypropyl methylcellulose,

(13) The process for production according to the above-mentioned (11), wherein the formulation in step b) is carried out by a compression formation and formation pressure in the compression formation is 3 to 14kN,

(14) The sustained-release preparation obtained by the process for production according to the above-mentioned (13), wherein the absolute hardness of the formulated substance obtained in step b) is 0.8 to 4.5N/$mm^2$,

(15) A controlled-release preparation having a controlled-release coating layer on a formulated substance containing a blood coagulation factor Xa inhibitor,

(16) The controlled-release preparation according to the above-mentioned (15), wherein the blood coagulation factor Xa inhibitor is 1-(1-{(2S)-3-[(6-chloronaphthalen-2-yl)sulfonyl]-2-hydroxypropanoyl}piperidin-4-yl)tetrahydropyrimidin-2(1H)-one, and

(17) The controlled-release preparation according to the above-mentioned (15), wherein the controlled-release coating layer is a controlled-release coating layer containing a pH-dependently soluble polymer.

Effects of the Invention

The sustained-release preparation, pharmaceutical composition and controlled-release preparation containing a FXa inhibitor of the present invention can release the FXa inhibitor over a long period and inhibit appropriately FXa activity. Therefore, in that the sustained-release preparation, pharmaceutical composition and controlled-release preparation have less side effects and an administration of once a day will suffice, they are useful as a medicine (for example, a prophylactic or therapeutic agent for thrombosis) excellent in convenience, compliance and safety.

Best Mode for Carrying Out the Invention

Herein, the "FXa inhibitor" is not particularly limited, as long as a compound has a FXa inhibitory action. For example, the compound disclosed in WO 96/16940, that is represented by the formula:

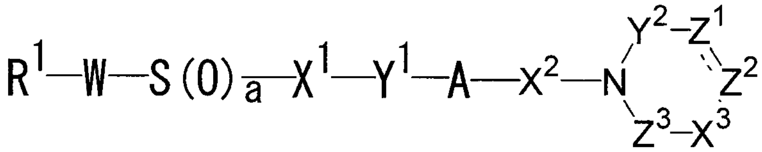

(I)

wherein, $R^1$ represents an optionally substituted cyclic hydrocarbon group or an optionally substituted heterocyclic group,
W represents a bond or an optionally substituted divalent chain hydrocarbon group,
a represents 0, 1, or 2,
$X^1$ represents an optionally substituted lower alkylene or an optionally substituted lower alkenylene,
$Y^1$ represents -C(O)-, -S(O)- or $-S(O)_2$,

A represents a piperazine ring which may be further substituted or a piperidine ring which may be further substituted,
$X^2$ represents a bond or an optionally substituted lower alkylene,
$Y^2$ represents -C(O)-, -S(O)- , $-S(O)_2-$ or $-C(=NR^7)-$ (wherein $R^7$ represents a hydrogen atom, an optionally substituted hydroxy group, a lower alkoxycarbonyl group or an acyl group),
$X^3$ represents an optionally substituted $C_{1-4}$ alkylene or an optionally substituted $C_{2-4}$ alkenylene, or when $X^3$ represents a $C_{2-4}$ alkenylene substituted with two alkyl groups, two alkyl groups may be bound to each other to form an aryl ring together with carbon atoms which they attach to,
$Z^3$ represents $-N(R^4)-$, -O- or a bond (wherein $R^4$ represents a hydrogen atom, an optionally substituted hydrocarbon group or an acyl group), ------ represents a single bond or a double bond, and when ------ represents a single bond, $Z^1$ represents $-C(R^2)(R^{2'})-$, $-N(R^2)-$ or -O-, and $Z^2$ represents $-C(R^3)(R^{3'})-$, $-N(R^3)-$, -O- or a bond (provided that, when $Z^1$ is -O-, $Z^2$ is other than -O-), and when ----- is a double bond, $Z^1$ represents $-C(R^2)=$ or a nitrogen atom, and $Z^2$ represents $=C(R^3)-$ or a nitrogen atom, each of $R^2$, $R^{2'}$, $R^3$ and $R^{3'}$ represents a hydrogen atom, an optionally substituted hydrocarbon group or an optionally substituted heterocyclic group, respectively, or each pair of $R^2$ and $R^3$, and $R^{2'}$ and $R^{3'}$ may be bound to each other respectively to form an optionally substituted ring, or a salt thereof are preferably used.

Specifically, as the compound represented by formula (I), preferred are 4-(1-{(2S)-3-[(6-chloronaphthalen-2-yl)sulfonyl]-2-hydroxypropanoyl}piperidin-4-yl)morpholin-3-one, 1-(4-{(2S)-3-[(6-chloronaphthalen-2-yl)sulfonyl]-2-hydroxypropanoyl}piperazin-1-yl)piperidin-2-one, 1-(1-{(2S)-3-[(6-chloronaphthalen-2-yl)sulfonyl]-2-hydroxypropanoyl} piperidin-4-yl)tetrahydropyrimidin-2(1H)-one, 1-(4-{(2S)-3-[(6-chloronaphthalen-2-yl)sulfonyl]-2-hydroxypropanoyl}piperazin-1-yl)tetrahydropyrimidin-2(1H)-one, 1-(1-{3-[(6-chloronaphthalen-2-yl)sulfonyl]-propanoyl)piperazin-4-yl)tetrahydropyrimidin-2(1H)-one, (2S)-3-[(6-chloronaphthalen-2-yl)sulfonyl]-1-(2-imino-1,4'-bipiperidin-1'-yl)-1-oxopropan-2-ol, 1'-{(2S)-3-[(6-chloronaphthalen-2-yl)sulfonyl]-2-hydroxypropanoyl}-1,4'-bipiperidin-2-one, and 2-(1-{(2S)-3-[(6-chloronaphthalen-2-yl)sulfonyl]-2-hydroxypropanoyl}piperidin-4-yl)isoindolin-1-one.

Among these, 1-(1-{(2S)-3-[(6-chloronaphthalen-2-yl)sulfonyl]-2-hydroxypropanoyl}piperidin-4-yl)tetrahydropyrimidin-2(1H)-one (hereinafter, referred to as Compound A occasionally) is more preferred.

Herein, the "sustained-release preparation" means a preparation wherein the "dissolution rate of drug from the preparation in the 30 minutes after initiation of test" is less than 85% when the Dissolution Test Method 2 (Paddle Method) of the Japanese Pharmacopoeia is conducted using a suitable test solution (900mL) under the condition of paddle rotation number of 100rpm. Here, as the test solution (dissolution medium), for example, a test solution is used wherein the drug concentration becomes to be 1/3 or less of saturation solubility of the drug when 100% of the drug in the preparation is dissolved in the test solution. In addition, as the test solution, those used conventionally in the formulation field, for example, water, buffer solution and the like are used.

Further, in this specification, the preparation wherein the dissolution rate of drug from the preparation in the 30 minutes after initiation of test is 85% or more when the Dissolution Test Method 2 (Paddle Method) of the Japanese Pharmacopoeia is conducted under the same condition as described above, is referred to as quick-release preparation.

The sustained-release preparation in the "sustained-release preparation comprising a FXa inhibitor and a hydrophilic polymer" of the present invention not only contains a FXa inhibitor and a hydrophilic polymer but also has to achieve the above-described dissolution rate of drug.

Herein, the hydrophilic polymer means a polymer that can control the release of FXa inhibitor by absorbing water to become hydrogel and diffusing FXa inhibitor contained in the preparation, or by itself being dissolved in water.

The viscosity of the hydrophilic polymer is, for example, as viscosity of 2% by weight aqueous solution (measurement temperature: 20°C), preferably 1 mPa·s-200000 mPa·s, more preferably 4 mPa·s-120000 mPa·s, further more preferably 4 mPa·s-5000 mPa·s. In the sustained-release preparation of the present invention, the release duration of FXa inhibitor from the preparation can be adjusted arbitrarily by adjusting the viscosity of the hydrophilic polymer to be used as a base material.

Specific examples of the hydrophilic polymer include hydroxypropyl celluloses (HPC) such as HPC-SSL (trade name, manufactured by NIPPON SODA CO., viscosity of 2% by weight aqueous solution at 20°C: 2.0-2.9 mPa·s), HPC-SL (trade name, manufactured by NIPPON SODA CO., viscosity of 2% by weight aqueous solution at 20°C: 3.0-5.9 mPa·s), HPC-L (trade name, manufactured by NIPPON SODA CO., viscosity of 2% by weight aqueous solution at 20°C: 6.0-10.0 mPa·s), HPC-M (trade name, manufactured by NIPPON SODA CO., viscosity of 2% by weight aqueous solution at 20°C: 150-400 mPa·s), HPC-H (trade name, manufactured by NIPPON SODA CO., viscosity of 2% by weight aqueous solution at 20°C: 1000-4000 mPa·s); hydroxypropyl methylcelluloses such as Metolose SB-4 (trade name, manufactured by Shin-Etsu Chemical CO., viscosity of 2% by weight aqueous solution at 20°C: about 4 mPa·s), TC-5RW (trade name, manufactured by Shin-Etsu Chemical CO., viscosity of 2% by weight aqueous solution at 20°C: about 6 mPa·s), TC-5S (trade name, manufactured by Shin-Etsu Chemical CO., viscosity of 2% by weight aqueous solution at 20°C: about 15 mPa·s), Metolose 60SH-50 (trade name, manufactured by Shin-Etsu Chemical CO., viscosity of 2% by weight aqueous solution at 20°C: about 50 mPa·s), Metolose 65SH-50 (trade name, manufactured by Shin-Etsu Chemical CO., viscosity of 2% by weight aqueous solution at 20°C: about 50 mPa·s), Metolose 90SH-100 (trade name, manufactured by Shin-Etsu Chemical CO., viscosity of 2% by weight aqueous solution at 20°C: about 100 mPa·s), Metolose 90SH-100SR (trade name, manufactured by Shin-Etsu Chemical CO., viscosity of 2% by weight aqueous solution at 20°C: about 100 mPa·s), Metolose 65SH-400 (trade name, manufactured by Shin-Etsu Chemical CO., viscosity of 2% by weight aqueous solution at 20°C: about 400 mPa·s), Metolose 90SH-400 (trade name, manufactured by Shin-Etsu Chemical CO., viscosity of 2% by weight aqueous solution at 20°C: about 400 mPa·s), Metolose 65SH-1500 (trade name, manufactured by Shin-Etsu Chemical CO., viscosity of 2% by weight aqueous solution at 20°C: about 1500 mPa·s), Metolose 60SH-4000 (trade name, manufactured by Shin-Etsu Chemical CO., viscosity of 2% by weight aqueous solution at 20°C: about 4000 mPa·s), Metolose 65SH-4000 (trade name, manufactured by Shin-Etsu Chemical CO., viscosity of 2% by weight aqueous solution at 20°C: about 4000 mPa·s), Metolose 90SH-4000 (trade name, manufactured by Shin-Etsu Chemical CO., viscosity of 2% by weight aqueous solution at 20°C: about 4000 mPa·s), Metolose 90SH-4000SR (trade name, manufactured by Shin-Etsu Chemical CO., viscosity of 2% by weight aqueous solution at 20°C: about 4000 mPa·s), Metolose 90SH-30000 (trade name, manufactured by Shin-Etsu Chemical CO., viscosity of 2% by weight aqueous solution at 20°C: about 30000 mPa·s), Metolose 90SH-100000 (trade name, manufactured by Shin-Etsu Chemical CO., viscosity of 2% by weight aqueous solution at 20°C: about 100000 mPa·s), Metolose 90SH-100000SR (trade name, manufactured by Shin-Etsu Chemical CO., viscosity of 2% by weight aqueous solution at 20°C: about 100000 mPa·s);
methylcelluloses such as Metolose SM15 (trade name, manufactured by Shin-Etsu Chemical CO.; viscosity: about 15 mPa·s, 2% by weight aqueous solution, 20°C), Metolose SM25 (trade name, manufactured by Shin-Etsu Chemical CO., viscosity of 2% by weight aqueous solution at 20°C: about 25 mPa·s), Metolose SM100 (trade name, manufactured by Shin-Etsu Chemical CO., viscosity of 2% by weight aqueous solution at 20°C: about 100 mPa·s), Metolose SM400 (trade name, manufactured by Shin-Etsu Chemical CO., viscosity of 2% by weight aqueous solution at 20°C: about 400 mPa·s), Metolose SM1500 (trade name, manufactured by Shin-Etsu Chemical CO., viscosity of 2% by weight aqueous solution at 20°C: about 1500 mPa·s), Metolose SM4000 (trade name, manufactured by Shin-Etsu Chemical CO., viscosity of 2% by weight aqueous solution at 20°C: about 4000 mPa·s), Metolose SM8000 (trade name, manufactured by Shin-Etsu Chemical CO., viscosity of 2% by weight aqueous solution at 20°C: about 8000 mPa·s), polyethylene oxides such as WSR N-12K (trade name, manufactured by Union Carbide Co., viscosity of 2% by weight aqueous solution at 20°C: 400-800 mPa·s), WSR N-60K (trade name, manufactured by Union Carbide Co., viscosity of 2% by weight aqueous solution at 20°C: 2000-4000 mPa·s), WSR 301 (trade name, manufactured by Union Carbide Co., viscosity of 1% by weight aqueous solution at 25°C: 1500-4500 mPa·s), WSR Coagulant (trade name, manufactured by Union Carbide Co., viscosity of 1% by weight aqueous solution at 25°C: 4500-7500 mPa·s), WSR 303 (trade name, manufactured by Union Carbide Co., viscosity of 1% by weight aqueous solution at 25°C: 7500-10000 mPa·s), WSR 308 (trade name, manufactured by Union Carbide Co., viscosity of 1% by weight aqueous solution at 25°C: 10000-15000 mPa·s),
sodium carboxymethyl celluloses such as Sunrose F-150MC (trade name, manufactured by Nippon Paper Industries Co., viscosity of 1% by weight aqueous solution at 25°C: 1200-1800 mPa·s), Sunrose F-300MC (trade name, manufactured by Nippon Paper Industries Co., viscosity of 1% by weight aqueous solution at 25°C: 2500-3000 mPa·s), Sunrose F-1000MC (trade name, manufactured by Nippon Paper Industries Co., viscosity of 1% by weight aqueous solution at 25°C: 8000-12000 mPa·s); and the like. These hydrophilic polymers may be used with mixing 2 or more at an appropriate rate.

In particular, two hydrophilic polymers, hydroxypropyl cellulose and hydroxymethyl propylcellulose are preferred to use by mixing.

The "sustained-release preparation comprising a FXa inhibitor and a hydrophilic polymer" of the present invention may further contain a pH-dependently soluble polymer. Here, as said pH-dependently soluble polymer, polymers similar to the pH-dependently soluble polymers used in the "preparation that releases FXa inhibitor in response to changes in the environmental pH" to be described below, are used, and the release duration of FXa inhibitor from the preparation can be adjusted arbitrarily by adjusting the amount of the pH-dependently soluble polymer.

The content of FXa inhibitor in the sustained-release preparation varies depending on the kind of FXa inhibitor, size of the preparation, and the like, and it is, for example, 1 to 90% by weight, preferably 5 to 80% by weight.

The content of the hydrophilic polymer in the sustained-release preparation varies depending on the content of FXa inhibitor, size of the preparation, kind of hydrophilic polymer and the like, and it is 5 to 90% by weight, preferably 10 to 80% by weight. More preferably, 20 to 50% by weight is preferred.

Examples of the dosage form of the sustained-release preparation in the present invention include oral preparations such as tablets, capsules (including microcapsules), granules, powders and the like; and parenteral preparations such as suppository (e.g., rectum suppository, vaginal suppository, etc.) and the like, and these can safely be administered orally or parenterally, respectively. Among these, oral preparations such as tablets (including those film-coated), capsules and granules are preferred, and in particular, tablets are most preferred.

The sustained-release preparation of the present invention can be produced by mixing FXa inhibitor and hydrophilic polymer, and formulating. Herein, the mixing and formulating can be conducted according to a conventional method in the preparation technology field. In addition, in the above mixing and/or formulating, pharmacologically acceptable carriers may be used.

Herein, the pharmacologically acceptable carriers include various organic or inorganic carrier substances conventionally used as preparation materials, for example, excipients, lubricants, binders, disintegrants and the like. Further, if necessary, preparation additives such as antiseptics, antioxidants, colorants, sweeteners and the like can be used.

Preferable examples of the excipients include lactose, white sugar, D-mannitol, D-sorbitol, starch, $\alpha$ starch, dextrin, crystalline cellulose, low substituted hydroxypropyl cellulose, carboxymethyl cellulose sodium, gum arabic, dextrin, pullulan, light silicic acid anhydride, synthetic aluminum silicate, magnesium aluminometasilicate, and the like.

Preferable examples of the lubricants include magnesium stearate, calcium stearate, talc, colloidal silica and the like.

Preferable examples of the binders include $\alpha$ starch, sucrose, gelatin, gum arabic, methylcellulose, carboxymethyl cellulose, carboxymethyl cellulose sodium, crystalline cellulose, white sugar, D-mannitol, trehalose, dextrin, pullulan, hydroxypropyl cellulose, hydroxypropyl methylcellulose, polyvinyl pyrrolidone, and the like.

Preferable examples of the disintegrants include lactose, white sugar, starch, carboxymethyl cellulose, carboxymethyl cellulose calcium, cross carmelose sodium, carboxymethylstarch sodium, light silicic acid anhydride, low substituted hydroxypropyl cellulose, and the like.

Preferable examples of the antiseptics include p-hydroxybenzoic esters, chlorobutanol, benzyl alcohol, phenethyl alcohol, dehydroacetic acid, sorbic acid and the like.

Preferable examples of the antioxidants include bisulfate, ascorbate, and the like.

Preferable examples of the colorants include water-soluble edible tar pigments (e.g., edible pigments such as edible Red No.2 and No.3, edible Yellow No.4 and No.5, edible Blue no.1 and No.2, and the like), water-insoluble lake pigments (e.g., aluminum salt of the above-described water-soluble edible tar pigments), natural pigments (e.g., $\beta$-carotene, chlorophyll, colcothar, yellow iron sesquioxide), and the like.

Preferable examples of the sweeteners include saccharin sodium, glycylrrhizin dipotassium, aspartame, stevia, and the like.

When the FXa inhibitor to be used for the sustained-release preparation of the present invention is basic, an organic acid may be added for the purpose of adjusting the dissolution behavior of the sustained-release preparation. Since the solubility of basic drugs is generally higher under acidic condition than under neutral condition, drug dissolution from the sustained-release preparation may vary depending on the ambient pH. In such case, the change of drug dissolution by the ambient pH can be reduced by using organic acid. Reducing the change of drug dissolution by ambient pH is extremely significant in order to obtain a uniform drug efficacy for various patients because in vivo pH may differ in various patients.

Examples of the organic acid include citric acid, tartaric acid, ascorbic acid, malic acid, fumaric acid, malonic acid, succinic acid, maleic acid, aspartic acid, glutamic acid and the like. Among these, fumaric acid, citric acid, tartaric acid, ascorbic acid and the like are preferred.

The content of the organic acid in the sustained-release preparation varies depending on the kind and content of FXa inhibitor, size of the preparation and the like, and it is, for example, 1 to 50% by weight, preferably 5 to 30% by weight.

The sustained-release preparation of the present invention is useful for prevention (including secondary prevention) of various arterial and venous thrombosis of human beings and animals, in particular mammals (for example, human being, monkey, cat, pig, horse, cattle, mouse, rat, guinea pig, dog, rabbit, and the like), for example, myocardial infarction, cerebral infarction, deep vein thrombosis, pulmonary thromboembolism or atherosclerotic obliterans, economy-class syndrome, thromboembolism during and post operation, cancer and the following disorders, or treatment of organ.

Brain:

Prevention or treatment of cerebral infarction, ischemic cerebrovascular disorder, thromboembolic stroke caused by atrial fibrillation, heart failure, valvular disease and heart valve replacement, acute ischemic cerebral apoplexy, acute stage cerebral thrombosis, cerebrovascular contraction after subarachnoid hemorrhage, Alzheimer's disease, transient ischemic attack (TIA), mixed dementia, cerebrovascular dementia, asymptomatic/multiple cerebral infarction, lacunar infarction and the like, prognosis improvement or secondary onset prevention of cerebral infarction, prevention or treatment of thrombus after an extracranial and intracranial arterial bypass operation, combination use or supplemental use with a thrombolytic agent against cerebral infarction (among them, ischemic cerebrovascular disorder), combination therapy with an anti-platelet drug such as aspirin in preventing onset of cerebral infarction.

Heart:

Prevention or treatment of acute coronary disease such as acute myocardial infarction, myocardial infarction, ischemic coronary disease, unstable angina, myocardiopathy, acute heart failure, congestive chronic heart failure, valvular disease and the like, prognosis improvement or secondary onset prevention of acute coronary disease such as

angina, prevention or treatment of thrombus formation after artificial valve or artificial heart replacement, prevention or treatment of vascular reocclusion and restenosis after coronary intervention such as stent indwelling or PTCA (percutaneous transluminal coronary angioplasty) or atherectomy, prevention or treatment of vascular reocclusion and restenosis after coronary bypass operation, combination use or supplemental use with a thrombolytic agent against acute coronary disease, combination therapy with an anti-platelet drug such as aspirin in preventing onset of myocardial infarction.

Periphery:

Prevention or treatment of deep vein thrombosis, chronic arterial obliterans, atherosclerotic obliterans, peripheral circulation failure such as Buerger's disease, peripheral circulation failure after frostbite, aneurysm, varix, adult respiratory distress syndrome, acute renal failure, chronic renal disease (e.g. diabetic nephropathy, chronic glumerular nephritis, IgA nephropathy etc.), diabetic circulation disorder, pain, nerve disorder, diabetic complication such as diabetic retinopathy and the like, prognosis improvement or secondary onset prevention of deep vein thrombosis, prevention or treatment of deep vein thrombosis or pulmonary thromboembolism after a joint operation including total hip arthroplasty (THA) or total knee arthroplasty (TKA), prevention or treatment of deep vein thrombosis or pulmonary thromboembolism after an orthopedic, plastic surgical or general surgical operation including a spine operation, prevention or treatment of thrombus after a peripheral vascular bypass operation or artificial vessel or vena cava filter indwelling, prevention or treatment of reocclusion and restenosis after stent indwelling or PTA (percutaneous transluminal angioplasty) or peripheral vascular intervention such as atherectomy, prevention or treatment of deep vein thrombosis or pulmonary thromboembolism accompanied with acute internal disease, combination use or supplemental therapy with a thrombolytic agent against deep vein thrombosis and pulmonary thromboembolism, combination therapy with an anti-platelet drug such as aspirin in therapy of peripheral circulation failure such as arteriosclerotic obliterans.

Others:

Prevention or treatment of pulmonary embolism, acute pulmonary embolism, economy class syndrome, thrombocytopenia or activation of blood coagulation system or complement activation caused by dialysis, thrombocytopenia on a major operation, thrombocytopenic purpura, disseminated intravascular coagulation syndrome (DIC) developed in a patient suffering from progression of arteriosclerosis or cancer metastasis or systemic inflammatory reaction syndrome (SIRS) or pancreatitis or cancer or leukemia or a major operation or sepsis or the like, various organ disorders such as liver function disorder caused by oligemia or ischemia or retention of blood, various organ failures caused by progression of shock or DIC (e.g. lung failure, liver failure, kidney failure, heart failure etc.), systemic lupus erythematosus, diffuse collagen disease, hyperthyroidism, puerperal palsy and the like, inhibition of rejective response on transplantation, organ protection or function improvement on transplantation, prevention of perfusion blood coagulation during blood extracorporeal circulation, substitute therapeutic use against development of thrombocytopenia caused by heparin administration, promotion of bedsore or wound healing, inhibition of activation of blood excessive coagulation reaction on various hormone supplement therapy, substitute therapeutic use for a patient resistant or contraindicative to a coumarin drug including warfarin, inhibition of activation of excessive coagulation reaction on administration of a blood preparation or a blood coagulation factor-containing preparation, and the like.

Among these, it is preferably used for preventing and treating ischemic cerebral infarction ( e.g. thromboembolic stroke, for example, thromboembolic stroke due to atrial fibrillation, and the like; and ischemic cerebral infarction caused by progression of atherosclerosis or activation of blood coagulation system), deep vein thrombosis or pulmonary thromboembolism, for example, deep vein thrombosis or pulmonary thromboembolism after a joint operation including total hip arthroplasty (THA) or total knee arthroplasty (TKA); or the secondary prevention of acute coronary syndrome (ACS) (in particular, myocardial infarction and the like).

Dose of the sustained-release preparation of the present invention differs depending on administration subject, administration route, a disease to be treated and the like, and for example, when it is orally administered to an adult patient with vein thrombosis, the dose per day for adult (body weight about 60 kg) is about 1 to 1000 mg, preferably about 3 to 500 mg, and more preferably about 10 to 350 mg as active ingredient FXa inhibitor. These may be administered in one or two divided doses.

The in vivo FXa inhibitor release duration of the sustained-release preparation of the present invention is 1 hour to 72 hours, preferably 8 hour to 36 hours.

It is preferable that the plasma concentration of FXa inhibitor is maintained at 1.25 nmol/mL or less (preferably, 0.001 nmol/mL to 1.25 nmol/mL) over 1 hour to 24 hours after administration. In addition, It is also preferable to decrease FXa activity at least 10% or more (preferably, 10% to 99%) than usual, and it is more preferable to decrease it 20% or more (20% to 70%) than usual.

The sustained-release preparation of the present invention can be used appropriately in combination with a

drug (hereinafter, abbreviated as a combined drug) such as an antithrombotic agent, an Alzheimer's disease treating drug (e.g. Avan, Calan etc.), a cholesterol treating drug (e.g. an HMG-CoA reductase inhibitor such as simvastatin, pravastatin etc.), a TG lowering drug (e.g. clofibrate etc.), an AII antagonist (e.g. candesartan cilexetil, losartan etc.), an anti-platelet drug (e.g. clopidogrel, abciximab, aspirin etc.), a Ca antagonist (e.g. Calslot, amlodipine etc.), an ACE inhibitor (e.g. enalapril, captopril etc.), a β blocker (e.g. metoprolol, carvedilol etc.) or an antiarrhythmic drug (e.g. procaine amide etc.). The combined drug may be a low-molecular compound, a high-molecular protein, a polypeptide, an antibody, or a vaccine. In this case, administration timing of the sustained-release preparation of the present invention and a combined drug is not limited particularly, and these may be administered to the administration subject simultaneously or administered at an interval. Further, the sustained-release preparation of the present invention and a combined drug may be administered as two kinds of preparations containing respective active ingredient, or may be formulated to administer as a single sustained-release preparation containing both active ingredients.

The dose of a combined drug can be selected appropriately on the basis of a dose clinically used. In addition, a combination ratio of the sustained-release preparation of the present invention and the combined drug can be selected appropriately depending on a subject to be administered, administration route, a disease to be treated, symptom, and a combination thereof. For example, when a subject to be administered is a human, 0.01 to 100 parts by weight of the combined drug may be used relative to 1 part by weight of the FXa inhibitor that is an active ingredient of the sustained-release preparation of the present invention.

Examples of the antithrombotic agent include heparins (e.g., heparin sodium, heparin calcium, dalteparin sodium), warfarins (e.g., warfarin potassium), antithrombin drugs (e.g., aragatroban), thrombolytic drugs (e.g. urokinase, tisokinase, alteplase, nateplase, monteplase, pamiteplase), antiplatelet drugs (e.g., ticlopidine hydrochloride, cilostazol, ethyl icosapentate, beraprost sodium, sarpogrelate hydrochloride) and the like.

The present invention further relates to a "pharmaceutical composition comprising a combination of preparations containing 2 or more FXa inhibitors that have a different release rate of FXa inhibitor".

Here, the "preparation containing FXa inhibitor" may be any one as long as it contains FXa inhibitor, and may be a sustained-release preparation or a quick-release preparation. In addition, control mechanism of FXa inhibitor release in the "preparation containing FXa inhibitor" is not particularly limited, and it may be any one of a preparation that releases FXa inhibitor from the preparation by a passive diffusion, a preparation that releases FXa inhibitor with erosion of the preparation, a preparation that releases FXa inhibitor in response to changes in the environmental pH, a preparation that releases FXa inhibitor by inner pressure caused by inside expansion of the preparation due to absorption of the environmental water, a preparation that releases rapidly FXa inhibitor by disintegration or dissolution, and the like.

Here, as the "preparation that releases FXa inhibitor from the preparation by a passive diffusion", for example, the above-mentioned sustained-release preparation of the present invention [preferably, matrix tablets using hydrophilic polymer (e.g., hydroxypropyl cellulose, hydroxypropyl methylcellulose, polyethylene oxide), matrix tablets using lipophilic base materials (e.g., carnauba wax, hardened castor oil, hardened rape seed oil, polyglycerin fatty acid ester), tablets or granules coated with sustained-release base materials (e.g., cellulose polymers such as ethylcellulose; acrylic acid copolymer such as aminoalkyl methacrylate copolymer RS [Eudragit RS (trade name, Degussa Co.)], ethyl acrylate-methyl methacrylate copolymer suspension [Eudragit NE (trade name, Degussa Co.)]), and the like are exemplified.

Examples of the "preparation that releases FXa inhibitor with erosion of the preparation" include capsules compounded with polyglycolated glycerides (e.g., Gelucire 50/13 (trade name, GATTEFOSSE Co.) and the like.

As the "preparation that releases FXa inhibitor in response to changes in the environmental pH", a controlled-release preparation containing a pH-dependently soluble substance is used, and it may be any dosage form as long as it is a controlled-release preparation containing a blood coagulation factor Xa inhibitor and a pH-dependently soluble polymer.

Preferably, for example, a controlled-release preparation comprising a formulated substance containing a blood coagulation factor Xa inhibitor, a hydrophilic polymer and a pH-dependently soluble polymer, a controlled-release preparation having a controlled-release coating layer comprising a pH-dependently soluble polymer on a formulated substance containing a blood coagulation factor Xa inhibitor, and the like are exemplified.

As the controlled-release preparation comprising a formulated substance containing a blood coagulation factor Xa inhibitor, a hydrophilic polymer and a pH-dependently soluble polymer, among the above-mentioned sustained-release preparation of the present invention, a preparation (for example, tablets, granules etc.) comprising further a substance that controls the lease pH-dependently in addition to a hydrophilic polymer (e.g., hydroxypropyl cellulose, hydroxypropyl methylcellulose, polyethylene oxide), and the like are exemplified.

As the controlled-release preparation having a controlled-release coating layer comprising a pH-dependently soluble polymer on a formulated substance containing a blood coagulation factor Xa inhibitor, for example, a controlled-release preparation (for example, tablets, granules or powders, or tablets or capsules compounded these controlled-release granules or powders, etc.) coated with a substance that controls the lease pH-dependently on a formulated substance containing a FXa inhibitor, and the like are exemplified.

In the present specification, the term "pH-dependently" refers to releasing a FXa inhibitor under the environment

of a certain pH or more. Examples of the pH-dependently soluble substance herein include hydroxypropyl methylcellulose phthalate, cellulose acetate phthalate, carboxymethyl ethyl cellulose, methyl methacrylate-methacrylic acid copolymer, methacrylic acid-ethyl acrylate copolymer, ethyl acrylate-methyl methacrylate-trimethylammoniumethyl methacrylate chloride copolymer, methyl methacrylate-ethyl acrylate copolymer, methacrylic acid-methyl acrylate-methyl methacrylate copolymer, hydroxypropyl cellulose acetate succinate, polyvinyl acetate phthalate and the like, and these may be used with combining 2 or more.

Examples of the "preparation that releases FXa inhibitor by inner pressure caused by inside expansion of the preparation due to absorption of the environmental water" include OROS system (trade name, ALZA) and the like.

Examples of the "preparation that releases rapidly FXa inhibitor by disintegration or dissolution" include a preparation obtained by mixing a FXa inhibitor and a pharmacologically acceptable carrier, followed by formulating the mixture. Here, examples of the pharmacologically acceptable carrier include the same as in the above-mentioned sustained-release preparation of the present invention. In addition, mixing and formulating can be conducted according to a method conventionally used in the preparation technology field.

The release controlling mechanism of the preparations containing a blood coagulation factor Xa inhibitor in the "pharmaceutical composition comprising a combination of preparations containing 2 or more blood coagulation factor Xa inhibitors that have a different release rate of a blood coagulation factor Xa inhibitor" of the present invention may be the same or different from each other as long as the release rates are different. The "preparations containing 2 or more FXa inhibitors" may be a single preparation or separate independent plural preparations. Here, as the single preparation, a single capsule wherein preparations containing 2 or more FXa inhibitors are encapsulated; a multilayer tablet (preferably, bilayer tablet) having plural release-controlling portions or a nucleated tablet, and the like are exemplified.

The pharmaceutical composition is preferred to be comprised of a combination of a sustained-release preparation containing FXa inhibitor and a quick-release preparation containing FXa inhibitor. By employing these combinations, an excellent FXa inhibitory action can be achieved over a long period immediately after the administration.

The content of FXa inhibitor in the preparation containing FXa inhibitor varies depending on the kind of FXa inhibitor, size of the preparation, and the like, and it is, for example, 1 to 90% by weight, preferably 5 to 80% by weight.

The dosage form of the preparation containing FXa inhibitor is the same as in the above-mentioned sustained-release preparation of the present invention. As the sustained-release preparation containing FXa inhibitor, the above-mentioned sustained-release preparation of the present invention is preferred.

The pharmaceutical composition of the present invention is low toxic and has a less side effect, and thus can be used as a prophylactic or therapeutic agent for various diseases to a mammal just as the above-mentioned sustained-release preparation of the present invention.

Administration form of the pharmaceutical composition of the present invention is not particularly limited as long as the preparations containing 2 or more FXa inhibitors are combined at the time of administration. Examples of such administration form include 1) administration as a single preparation containing 2 or more FXa inhibitors, 2) simultaneous administration as plural preparations of preparations containing 2 or more FXa inhibitors, 3) sequential and intermittent administration as plural preparations of preparations containing 2 or more FXa inhibitors, and the like.

Dose of the pharmaceutical composition of the present invention differs depending on administration subject, administration route, a disease to be treated and the like, and administration with the same dose as FXa inhibitor as in the above-mentioned sustained-release preparation of the present invention is preferred.

The pharmaceutical composition of the present invention may be used in combination with a combined drug similar to in the above-mentioned sustained-release preparation of the present invention.

In addition, the pharmaceutical composition of the present invention is preferred to be administered at the time when an in vivo action of FXa inhibitor can be obtained continuously at least between before eating and about 2 hours after eating (preferably 4 hours after eating).

The present invention further relates to "a controlled-release preparation containing a blood coagulation factor Xa inhibitor, which can maintain a plasma level of the blood coagulation factor Xa inhibitor at 1.25 nmol/mL or less over one hour to 12 hours after the administration and can reduce the FXa activity in plasma by 10% or more than usual over one hour to 12 hours after the administration".

Here, the plasma in the "plasma level of the blood coagulation factor Xa inhibitor" and the "FXa activity in plasma" means a plasma of peripheral venous blood. FXa activity and decreasing rate thereof may differ depending on the kind of plasma (for example, plasma of vein, artery or portal vein), preferred is a controlled-release preparation that can reduce the FXa activity in plasma of peripheral venous blood by 10% or more (preferably 10% to 99%) than usual, more preferably, 20% or more (preferably 20% to 70%).

In addition, a controlled-release preparation is preferred that can maintain a plasma level of the blood coagulation factor Xa inhibitor at 1.25 nmol/mL or less, preferably, 0.001nmol/mL to 1.25 nmol/mL.

The FXa activity in plasma can be measured by, for example, similar methods to that described in Experimental Example 1 of WO 96/16940.

As the controlled-release preparation containing a FXa inhibitor of the present invention, among the above-mentioned preparations containing a FXa inhibitor of the present invention, a preparation in which the release of FXa inhibitor is controlled is exemplified.

As such preparation, the above-mentioned sustained-release preparation of the present invention is preferred. Further, among the above-mentioned pharmaceutical composition of the present invention, the "pharmaceutical composition comprising a combination of a sustained-release preparation containing FXa inhibitor and a quick-release preparation containing FXa inhibitor" is also preferred.

The controlled-release preparation containing FXa inhibitor of the present invention is low toxic and has a less side effect, and thus can be used as a prophylactic or therapeutic agent for various diseases to a mammal just as the above-mentioned sustained-release preparation of the present invention.

Dose of the controlled-release preparation containing FXa inhibitor of the present invention differs depending on administration subject, administration route, a disease to be treated and the like, and administration with the same dose as FXa inhibitor as in the above-mentioned sustained-release preparation of the present invention is preferred.

In addition, the controlled-release preparation containing FXa inhibitor of the present invention is preferred to be administered at the time when an in vivo action of FXa inhibitor can be obtained continuously at least between before eating and about 2 hours after eating (preferably 4 hours after eating).

The controlled-release preparation containing FXa inhibitor of the present invention may be used in combination with a combined drug similar to in the above-mentioned sustained-release preparation of the present invention.

In addition, the present invention relates to a process for production of a sustained-release preparation wherein defects of formulation in compression formulation are improved, and a formulated substance obtained by the process for production.

The process for production of a sustained-release preparation wherein defects of formulation in compression formulation are improved, of the present invention refers to a process for production comprising the following step a) and b).

Step a) is a step for granulating a mixture comprising an active ingredient and mannitol with spraying an aqueous solution of hydroxypropyl cellulose, and
step b) is a step for formulating a mixture comprising the granulated material obtained in step a) and 2 or more hydrophilic polymers to obtain a formulated substance.

The active ingredient to be used in step a) is not particularly limited, and blood coagulation factor Xa inhibitors are preferred, and preferably used is the same compounds as the blood coagulation factor Xa inhibitor used in the above-mentioned sustained-release preparation containing a blood coagulation factor Xa inhibitor and a hydrophilic polymer.

As the hydroxypropyl cellulose in the aqueous solution of hydroxypropyl cellulose to be used in step a), the hydroxypropyl cellulose (HPC) exemplified as a specific example of the above-mentioned hydrophilic polymer is used, among these, HPC-SSL, HPC-SL, and HPC-L is preferred.

The concentration of hydroxypropyl cellulose is preferred to be about 3 to 8%.

The 2 or more hydrophilic polymers to be used in step b) is not particularly limited, and preferred is the hydrophilic polymer that is used in the above-mentioned sustained-release preparation containing a blood coagulation factor Xa inhibitor and a hydrophilic polymer, and in particular, two of hydroxypropyl cellulose (HPC) and hydroxymethyl propylcellulose (HPMC) are preferred to use. The compounding rate of the 2 or more hydrophilic polymers is preferably, for example, 1 to 50% by weight, more preferably, 5 to 30% by weight as total amount of two hydrophilic polymers in the preparation.

The method in step b) for formulating a mixture comprising the granulated material obtained in step a) and 2 or more hydrophilic polymers is not particularly limited, and preferably, compression formulation is exemplified. The compression formulation can be carried out according to a method conventionally used in the preparation technology field, and formulation pressure in the compression formulation is preferably 3 to 14 kN.

Further, as the process for production of the sustained-release preparation of the present invention, in addition to step a) to b), a step for film coating the sustained-release preparation obtained in step b) may be included as step c).

As the film coating method in step c), it is preferable to produce a film-coated tablet by coating with about 2 to 6% to the weight of the core tablet obtained in step b) using, for example, Opadry aqueous suspension as coating agent.

The formulated substance to be obtained in the process for production of the present invention is preferably a formulated substance wherein the absolute hardness of the formulated substance obtained in step b) is 0.8 to 4.5 $N/mm^2$, and more preferably a formulated substance wherein the absolute hardness of the formulated substance obtained in step b) is 2.0 to 3.5 $N/mm^2$.

In addition, the absolute hardness is hardness per unit area, and defined by the following formula.

$$\text{absolute hardness (N/mm}^2\text{)} = \text{hardness (N)/(thickness (mm) x diameter (mm))}$$

Mixing, granulating, formulating, and coating to be conducted in these steps can be carried out according to a method conventionally used in the preparation technology field.

By the production method of the sustained-release preparation obtained in the process for production of the present invention, it becomes possible to decrease formulation obstacles in compression formulation and provide a sustained-release preparation having a good productivity since enough intensity can be provided with low formulation pressure by combining 2 or more hydrophilic polymers to enhance binding force.

Examples

The present invention is further detailed in the following Examples, Reference Examples and Experiments, which are not intended to restrict the present invention and can be modified without departing the scope of the present invention.

In addition, Compound A to be used in the following Examples, Reference Examples and Experiments can be synthesized according to the method described in Example 11 of WO 96/16940.

[Example 1]

Compound A (0.35g), Hypromellose 2208 (Metolose 90SH-4000, Shin-Etsu Chemical CO.) 4.2g, crystalline cellulose 2.1g, lactose 3.980g, and magnesium stearate 0.053g were mixed with pestle on a mortar. The obtained blended granule was compressed into tablets each weighing 300 mg with 9.5 mm diameter punch under tableting pressure 14 kN using universal testing machine (Autograph AG-I, Shimadzu) to obtain tablets.

[Example 2]

Compound A (2437g), mannitol (1499g) and crystalline cellulose (720g) were charged in a fluidized-bed granulator (FD-5S, Powrex Corp.), and granulated with spraying 2400g of 6% aqueous solution of hydroxypropylcellulose (HPC-L, NIPPON SODA CO.) to obtain granules. The resulting granules were charged in a granulator (powder mill, Showakagaku Kikai) to obtain milled granules. Then, the milled granules (2100g), Hypromellose 2208 (Metolose 90SH-4000, Shin-Etsu Chemical CO.) 861.0g, hydroxypropylcellulose (HPC-L) 252.0g, crystalline cellulose 315.0g, and magnesium stearate 42.0g were mixed with a blender(tumbler blender, Showakagaku Kikai). The obtained blended granules were compressed into tablets each weighing 340 mg with 9.0 mm diameter punch under tableting pressure 10 kN using a rotary tableting machine (Correct 19K, Kikusui Seisakusho) to obtain core tablets. The resulting core tablets were subjected to coating by about 4% to the weight of the core tablet with 10% aqueous suspension of Opadry red 03F45055 (Nippon Colorcon) using a coating machine (Driacoater DRC-500, Powrex Corp.) to obtain film-coated tablets.

[Example 3]

Milled granules (2100g) prepared similarly as in Example 2, Hypromellose 2208 (Metolose 90SH-100SR, Shin-Etsu Chemical CO.) 756.0g, Hypromellose 2208 (Metolose 90SH-4000SR, Shin-Etsu Chemical CO.) 105.0g, hydroxypropylcellulose (HPC-H, NIPPON SODA CO.) 252.0g, crystalline cellulose 315.0g, and magnesium stearate 42.0g were mixed with a blender (tumbler blender, Showakagaku Kikai). The obtained blended granules were compressed into tablets each weighing 340 mg with 9.0 mm diameter punch under tableting pressure 10 kN using a rotary tableting machine (Correct 19K, Kikusui Seisakusho) to obtain core tablets. The resulting core tablets were subjected to coating by about 4% to the weight of the core tablet with 10% aqueous suspension of Opadry red 03F45055 (Nippon Colorcon) using a coating machine (Driacoater DRC-500, Powrex Corp.) to obtain film-coated tablets.

[Example 4]

Milled granules (2100g) prepared similarly as in Example 2, Hypromellose 2208 (Metolose 90SH-4000SR, Shin-Etsu Chemical CO.) 861.0g, hydroxypropylcellulose (HPC-H) 252.0g, crystalline cellulose 315.0g, and magnesium stearate 42.0g were mixed with a blender (tumbler blender, Showakagaku Kikai). The obtained blended granules were compressed into tablets each weighing 340 mg with 9.0 mm diameter punch under tableting pressure 10 kN using a

rotary tableting machine (Correct 19K, Kikusui Seisakusho) to obtain core tablets. The resulting core tablets were subjected to coating by about 4% to the weight of the core tablet with 10% aqueous suspension of Opadry red 03F45055 (Nippon Colorcon) using a coating machine (Driacoater DRC-500, Powrex Corp.) to obtain film-coated tablets.

[Example 5]

The hardness of the tablet obtained by the hereinafter-described production method with formulation pressure of 6 kN was measured. The formulation prescription and resuls are shown in Table 1.

[Table 1]

| (Granulation) | 5-1 | 5-2 | 5-3 | 5-4 | 5-5 |
|---|---|---|---|---|---|
| Compound A | 100 | 100 | 100 | 100 | 100 |
| D-mannitol | 64 | 64 | 64 | 64 | 64 |
| crystalline cellulose | 30 | 30 | 30 | 30 | 30 |
| HPC-L | 6 | 6 | 6 | 6 | 6 |
| (formulation) | | | | | |
| HPMC 90SH-4000 | 112 | 82 | 82 | 68 | 68 |
| crystalline cellulose PH302 | - | 30 | - | - | - |
| crystalline cellulose KG802 | - | - | 30 | - | - |
| HPC-M | 24 | - | - | 68 | - |
| HPC-H | - | 24 | 24 | - | 68 |
| magnesium stearate | 4 | 4 | 4 | 4 | 4 |
| Total (mg) | 340 | 340 | 340 | 340 | 340 |
| absolute hardness ($N/mm^2$) | 2.1 | 2.1 | 2.4 | 2.4 | 2.5 |

[Example 5-1]

Compound A (125g), mannitol (80g) and crystalline cellulose (PH-101, Asahi Kasei Chemicals Corp.) 37.5g were charged in a fluidized-bed granulator (LAB-1, Powrex Corp.), and granulated with spraying 235g of 3% aqueous solution of hydroxypropylcellulose (HPC-L, NIPPON SODA CO.) to obtain granules. Then, the granules (4g), Hypromellose 2208 (Metolose 90SH-4000SR, Shin-Etsu Chemical CO.) 2.24g, hydroxypropylcellulose (HPC-M, NIPPON SODA CO.) 0.48g, and magnesium stearate 0.08g were mixed. The obtained blended granule was compressed into tablets each weighing 340 mg with 10 mm diameter punch under tableting pressure 6 kN and formulation speed 10 mm/minute using universal testing machine (Autograph AG-I, Shimadzu) to obtain tablets.

[Example 5-2]

Compound A (125g), mannitol (80g) and crystalline cellulose (PH-101, Asahi Kasei Chemicals Corp.) 37.5g were charged in a fluidized-bed granulator (LAB-1, Powrex Corp.), and granulated with spraying 235g of 3% aqueous solution of hydroxypropylcellulose (HPC-L, NIPPON SODA CO.) to obtain granules. Then, the granules (4g), Hypromellose 2208 (Metolose 90SH-4000SR, Shin-Etsu Chemical CO.) 1.64g, crystalline cellulose (PH-302, Asahi Kasei Chemicals Corp.) 0.6g, hydroxypropylcellulose (HPC-H, NIPPON SODA CO.) 0.48g and magnesium stearate 0.08g were mixed. The obtained blended granule was compressed into tablets each weighing 340 mg with 10 mm diameter punch under tableting pressure 6 kN and formulation speed 10 mm/minute using universal testing machine (Autograph AG-I, Shimadzu) to obtain tablets.

[Example 5-3]

Compound A (125g), mannitol (80g) and crystalline cellulose (PH-101, Asahi Kasei Chemicals Corp.) 37.5g were charged in a fluidized-bed granulator (LAB-1, Powrex Corp.), and granulated with spraying 235g of 3% aqueous solution of hydroxypropylcellulose (HPC-L, NIPPON SODA CO.) to obtain granules. Then, the granules (4g), Hypromellose 2208 (Metolose 90SH-4000SR, Shin-Etsu Chemical CO.) 1.64g, crystalline cellulose (KG-802, Asahi Kasei Chemicals Corp.) 0.6g, hydroxypropylcellulose (HPC-H, NIPPON SODA CO.) 0.48g, and magnesium stearate 0.08g were mixed. The obtained blended granule was compressed into tablets each weighing 340 mg with 10 mm diameter punch

under tableting pressure 6 kN and formulation speed 10 mm/minute using universal testing machine (Autograph AG-I, Shimadzu) to obtain tablets.

[Example 5-4]

Compound A (125g), mannitol (80g) and crystalline cellulose (PH-101, Asahi Kasei Chemicals Corp.) 37.5g were charged in a fluidized-bed granulator (LAB-1, Powrex Corp.), and granulated with spraying 235g of 3% aqueous solution of hydroxypropylcellulose (HPC-L, NIPPON SODA CO.) to obtain granules. Then, the granules (4g), Hypromellose 2208 (Metolose 90SH-4000SR, Shin-Etsu Chemical CO.) 1.36g, hydroxypropylcellulose (HPC-M, NIPPON SODA CO.) 1.36g, and magnesium stearate 0.08g were mixed. The obtained blended granule was compressed into tablets each weighing 340 mg with 10 mm diameter punch under tableting pressure 6 kN and formulation speed 10 mm/minute using universal testing machine (Autograph AG-I, Shimadzu) to obtain tablets.

[Example 5-5]

Compound A (125g), mannitol (80g) and crystalline cellulose (PH-101, Asahi Kasei Chemicals Corp.) 37.5g were charged in a fluidized-bed granulator (LAB-1, Powrex Corp.), and granulated with spraying 235g of 3% aqueous solution of hydroxypropylcellulose (HPC-L, NIPPON SODA CO.) to obtain granules. Then, the granules (4g), Hypromellose 2208 (Metolose 90SH-4000SR, Shin-Etsu Chemical CO.) 1.36g, hydroxypropylcellulose (HPC-H, NIPPON SODA CO.) 1.36g, and magnesium stearate 0.08g were mixed. The obtained blended granule was compressed into tablets each weighing 340 mg with 10 mm diameter punch under tableting pressure 6 kN and formulation speed 10 mm/minute using universal testing machine (Autograph AG-I, Shimadzu) to obtain tablets.

[Example 6]

Step 1) Compound A (125g), mannitol (80g) and crystalline cellulose (PH-101, Asahi Kasei Chemicals Corp., 37.5g) were charged in a fluidized-bed granulator (LAB-1, Powrex Corp.), and granulated with spraying 235g of 3% aqueous solution of hydroxypropylcellulose (HPC-L, NIPPON SODA CO.) to obtain granules. Then, the granules 1.2g, cross carmelose sodium (Ac-Di-Sol, Asahi Kasei Chemicals Corp.) 0.068g and magnesium stearate 0.014g were mixed. The obtained blended granule was compressed into tablets each weighing 60 mg with 5 mm diameter punch under tableting pressure 6 kN and formulation speed 10 mm/minute using universal testing machine (Autograph AG-I, Shimadzu) to obtain tablets. Step 2) The granules 2.17g, Hypromellose 2208 (Metolose 90SH-100SR, Shin-Etsu Chemical CO.) 1.27g, crystalline cellulose (KG-802, Asahi Kasei Chemicals Corp.) 0.47g, hydroxypropylcellulose (HPC-L, NIPPON SODA CO.) 0.37g, and magnesium stearate 0.05g were mixed.
Step 3) The blended granule 140 mg obtained in step 2) was laid inside a 9 mm diameter mortar for tabletting, the tablet obtained in step 1) was put at the center of the mortar, and the blended granule 139 mg obtained in step 2) was added thereto, then the resulting stuffs were compressed into tablet with 9 mm diameter punch under tableting pressure 6 kN and formulation speed 10 mm/minute using universal testing machine (Autograph AG-I, Shimadzu) to obtain a nucleated tablet.

[Example 7]

Step 1) Compound A (125g), mannitol (80g) and crystalline cellulose (PH-101, Asahi Kasei Chemicals Corp., 37.5g) were charged in a fluidized-bed granulator (LAB-1, Powrex Corp.), and granulated with spraying 235g of 3% aqueous solution of hydroxypropylcellulose (HPC-L, NIPPON SODA CO.) to obtain granules. Then, the granules 1.2g, cross carmelose sodium (Ac-Di-Sol, Asahi Kasei Chemicals Corp.) 0.068g and magnesium stearate 0.014g were mixed. The obtained blended granule was compressed into tablets each weighing 60 mg with 5 mm diameter punch under tableting pressure 6 kN and formulation speed 10 mm/minute using universal testing machine (Autograph AG-I, Shimadzu) to obtain tablets.
Step 2) The granules 2.17g, Hypromellose 2208 (Metolose 90SH-100SR, Shin-Etsu Chemical CO.) 0.89g, crystalline cellulose (KG-802, Asahi Kasei Chemicals Corp.) 0.90g, hydroxypropylcellulose (HPC-L, NIPPON SODA CO.) 0.26g, and magnesium stearate 0.06g were mixed.
Step 3) The blended granule 140 mg obtained in step 2) was laid inside a 9 mm diameter mortar for tabletting, the tablet obtained in step 1) was put at the center of the mortar, and the blended granule 139 mg obtained in step 2) was added thereto, then the resulting stuffs were compressed into tablet with 9 mm diameter punch under tableting pressure 6 kN and formulation speed 10 mm/minute using universal testing machine (Autograph AG-I, Shimadzu) to obtain a nucleated tablet.

[Example 8]

Step 1) Compound A 9.0g, purified sucrose 59.6g, corn starch 80.0g and low substituted hydroxypropyl cellulose 40.0g were mixed well to prepare a dusting powder. 110g of white sugar-starch spheres (trade name: Nonpareil-101, Freund Industrial Co., Ltd.) were charged in a centrifugal fluid-bed granulator (CF-160, Freund Industrial Co., Ltd.), and the above dusting powder was coated on the spheres with spraying an aqueous solution (2 w/w%) of hydroxypropyl cellulose, thereby producing spherical granules. The spherical granules were dried at 40°C for 16 hours under vacuum and passed through a round sieve to obtain granules of 710μm-1180μm.
Step 2) Methacrylic acid copolymer S (23.7g), methacrylic acid copolymer L (7.9g) and triethyl citrate (3.2g) were dissolved in a mixed solution of purified water (45.4g) and absolute ethanol (408.3g), and talc (15.8g) was dispersed into the resulting solution to prepare a coating solution. The granules (70g) obtained in step 1) were coated with the above coating solution using an agitation fluidized bed granulator (SPIR-A-FLOW, Freund Industrial Co., Ltd.) under the condition of inlet air temperature: 35°C, rotor revolution speed: 80 rpm, coating solution spray rate: 2.5 g/minute and spray air pressure: 1.0 kg/cm$^2$, thereby obtaining pH-dependently soluble controlled granules. The obtained spherical granules were passed through a round sieve to give controlled-release granules of 850μm-1400μm. Then, the spherical granules were dried at 40°C for 16 hrs under vacuum.

[Reference Example 1]

Compound A (1312g), mannitol (11320g) and crystalline cellulose (1560g) are charged in a fluidized-bed granulator (FD-S2, Powrex Corp.), and granulated with spraying 7800g of 6% aqueous solution of hydroxypropylcellulose (HPC-L) to obtain granules. The resulting granules are charged in a granulator (powder mill P-3, Showakagaku Kikai) to obtain milled granules. To two batches (25150g) of the obtained milled granules, cross carmelose sodium (1338g) and magnesium stearate (267.6g) are added, and mixed with a blender (tumbler blender, Showakagaku Kikai). The resulting blended granule is compressed into tablets each weighing 120 mg with 6.5 mm diameter punch under tableting pressure 6 kN using a rotary tableting machine (Aquarius 36K, Kikusui Seisakusho) to obtain core tablets. The resulting core tablets are subjected to coating by 5 mg per core tablet with aqueous suspension (concentration of solid ingredients: 10%) prepared by dissolving in purified water Opadry red 03F45055 (Nippon Colorcon) which is a premixture of Hypromellose 2910, Macrogol 6000, titanium dioxide and red ferric oxide, using a coating machine (HCF-100F, manufactured by Freund Industrial Co.) to obtain 195000 film-coated tablets containing 10 mg of Compound A per tablet and having the following prescription.

Film-coated Tablet Prescription (composition per tablet):

| Composition | Compounded Amount (mg) |
|---|---|
| (1) Compound A | 10.0 |
| (2) D-mannitol | 87.2 |
| (3) crystalline cellulose | 12.0 |
| (4) cross carmelose sodium | 6.0 |
| (5) hydroxypropylcellulose | 3.6 |
| (6) magnesium stearate | 1.2 |
| (7) Hypromellose 2910 | 3.67 |
| (8) Macrogol 6000 | 0.83 |
| (9) titanium dioxide | 0.42 |
| (10) red ferric oxide | 0.08 |
| Total | 125.0 |

[Reference Example 2]

Compound A (1312g), lactose (11320g) and corn starch (1560g) are charged in a fluidized-bed granulator (FD-S2, Powrex Corp.), and granulated with spraying 7800g of 6% aqueous solution of Hypromellose 2910 (TC-5) to obtain granules. The resulting granules are charged in a granulator (powder mill P-3, Showakagaku Kikai) to obtain milled granules. To two batches (25150g) of the obtained milled granules, crosspovidon (1338g) and magnesium stearate (267.6g) are added, and mixed with a blender (tumbler blender, Showakagaku Kikai). The resulting blended granule is

compressed into tablets each weighing 120 mg with 6.5 mm diameter punch under tableting pressure 6 kN using a rotary tableting machine (Aquarius 36K, Kikusui Seisakusho) to obtain core tablets. The resulting core tablets are subjected to coating by 5 mg per core tablet with aqueous suspension (concentration of solid ingredients: 10%) prepared by dissolving in purified water Opadry red 03F45055 (Nippon Colorcon) which is a premixture of Hypromellose 2910, Macrogol 6000, titanium dioxide and red ferric oxide, using a coating machine (HCF-100F, manufactured by Freund Industrial Co.) to obtain 195000 film-coated tablets containing 10 mg of Compound A per tablet and having the following prescription.

Film-coated Tablet Prescription (composition per tablet):

| Composition | Compounded Amount (mg) |
|---|---|
| (1) Compound A | 10.0 |
| (2) lactose | 87.2 |
| (3) corn starch | 12.0 |
| (4) crosspovidon | 6.0 |
| (5) Hypromellose 2910 | 3.6 |
| (6) magnesium stearate | 1.2 |
| (7) Hypromellose 2910 | 3.67 |
| (8) Macrogol 6000 | 0.83 |
| (9) titanium dioxide | 0.42 |
| (10) red ferric oxide | 0.08 |
| Total | 125.0 |

[Reference Example 3]

Compound A (1312g), lactose (9088g) and crystalline cellulose (3900g) are charged in a fluidized-bed granulator (FD-S2, Powrex Corp.), and granulated with spraying 7800g of 4% aqueous solution of hydroxypropylcellulose (HPC-L) to obtain granules. The resulting granules are charged in a granulator (powder mill P-3, Showakagaku Kikai) to obtain milled granules. To two batches (25070g) of the obtained milled granules, carboxymethylstarch sodium (1338g), light silicic acid anhydride (89.20g) and magnesium stearate (267.6g) are added, and mixed with a blender (tumbler blender, Showakagaku Kikai). The resulting blended granule is compressed into tablets each weighing 120 mg with 6.5 mm diameter punch under tableting pressure 6 kN using a rotary tableting machine (Aquarius 36K, Kikusui Seisakusho) to obtain core tablets. The resulting core tablets are subjected to coating by 5 mg per core tablet with aqueous suspension (concentration of solid ingredients: 10%) prepared by dissolving in purified water Opadry red 03F45055 (Nippon Colorcon) which is a premixture of Hypromellose 2910, Macrogol 6000, titanium dioxide and red ferric oxide, using a coating machine (HCF-100F, manufactured by Freund Industrial Co.) to obtain 195000 film-coated tablets containing 10 mg of Compound A per tablet and having the following prescription.

Film-coated Tablet Prescription (composition per tablet):

| Composition | Compounded Amount (mg) |
|---|---|
| (1) Compound A | 10.0 |
| (2) lactose | 70.0 |
| (3) crystalline cellulose | 30.0 |
| (4) carboxymethylstarch sodium | 6.0 |
| (5) hydroxypropylcellulose | 2.4 |
| (6) light silicic acid anhydride | 0.4 |
| (7) magnesium stearate | 1.2 |
| (8) Hypromellose 2910 | 3.67 |
| (9) Macrogol 6000 | 0.83 |
| (10) titanium dioxide | 0.42 |

(continued)

| Composition | Compounded Amount (mg) |
|---|---|
| (11) red ferric oxide | 0.08 |
| Total | 125.0 |

[Reference Example 4]

Compound A (2624g), mannitol (10010g) and crystalline cellulose (1560g) are charged in a fluidized-bed granulator (FD-S2, Powrex Corp.), and granulated with spraying 7800g of 6% aqueous solution of hydroxypropylcellulose (HPC-L) to obtain granules. The resulting granules are charged in a granulator (powder mill P-3, Showakagaku Kikai) to obtain milled granules. To two batches (25150g) of the obtained milled granules, cross carmelose sodium (1338g) and magnesium stearate (267.6g) are added, and mixed with a blender (tumbler blender, Showakagaku Kikai). The resulting blended granule is compressed into tablets each weighing 120 mg with 6.5 mm diameter punch under tableting pressure 6 kN using a rotary tableting machine (Aquarius 36K, Kikusui Seisakusho) to obtain core tablets. The resulting core tablets are subjected to coating by 5 mg per core tablet with aqueous suspension (concentration of solid ingredients: 10%) prepared by dissolving in purified water Opadry red 03F45055 (Nippon Colorcon) which is a premixture of Hypromellose 2910, Macrogol 6000, titanium dioxide and red ferric oxide, using a coating machine (HCF-100F, manufactured by Freund Industrial Co.) to obtain 195000 film-coated tablets containing 20 mg of Compound A per tablet and having the following prescription.

Film-coated Tablet Prescription (composition per tablet):

| Composition | Compounded Amount (mg) |
|---|---|
| (1) Compound A | 20.0 |
| (2) D-mannitol | 77.2 |
| (3) crystalline cellulose | 12.0 |
| (4) cross carmelose sodium | 6.0 |
| (5) hydroxypropylcellulose | 3.6 |
| (6) magnesium stearate | 1.2 |
| (7) Hypromellose 2910 | 3.67 |
| (8) Macrogol 6000 | 0.83 |
| (9) titanium dioxide | 0.42 |
| (10) red ferric oxide | 0.08 |
| Total | 125.0 |

[Reference Example 5]

Compound A (2624g), lactose (10010g) and corn starch (1560g) are charged in a fluidized-bed granulator (FD-S2, Powrex Corp.), and granulated with spraying 7800g of 6% aqueous solution of Hypromellose (TC-5) to obtain granules. The resulting granules are charged in a granulator (powder mill P-3, Showakagaku Kikai) to obtain milled granules. To two batches (25150g) of the obtained milled granules, crosspovidon (1338g) and magnesium stearate (267.6g) are added, and mixed with a blender (tumbler blender, Showakagaku Kikai). The resulting blended granule is compressed into tablets each weighing 120 mg with 6.5 mm diameter punch under tableting pressure 6 kN using a rotary tableting machine (Aquarius 36K, Kikusui Seisakusho) to obtain core tablets. The resulting core tablets are subjected to coating by 5 mg per core tablet with aqueous suspension (concentration of solid ingredients: 10%) prepared by dissolving in purified water Opadry red 03F45055 (Nippon Colorcon) which is a premixture of Hypromellose 2910, Macrogol 6000, titanium dioxide and red ferric oxide, using a coating machine (HCF-100F, manufactured by Freund Industrial Co.) to obtain 195000 film-coated tablets containing 20 mg of Compound A per tablet and having the following prescription.

Film-coated Tablet Prescription (composition per tablet):

| Composition | Compounded Amount (mg) |
| --- | --- |
| (1) Compound A | 20.0 |
| (2) lactose | 77.2 |
| (3) corn starch | 12.0 |
| (4) crosspovidon | 6.0 |
| (5) Hypromellose 2910 | 3.6 |
| (6) magnesium stearate | 1.2 |
| (7) Hypromellose 2910 | 3.67 |
| (8) Macrogol 6000 | 0.83 |
| (9) titanium dioxide | 0.42 |
| (10) red ferric oxide | 0.08 |
| Total | 125.0 |

[Reference Example 6]

Compound A (2624g), lactose (8166g) and crystalline cellulose (3510g) are charged in a fluidized-bed granulator (FD-S2, Powrex Corp.), and granulated with spraying 7800g of 4% aqueous solution of hydroxypropylcellulose (HPC-L) to obtain granules. The resulting granules are charged in a granulator (powder mill P-3, Showakagaku Kikai) to obtain milled granules. To two batches (25070g) of the obtained milled granules, carboxymethylstarch sodium (1338g), light silicic acid anhydride (89.20g) and magnesium stearate (267.6g) are added, and mixed with a blender (tumbler blender, Showakagaku Kikai). The resulting blended granule is compressed into tablets each weighing 120 mg with 6.5 mm diameter punch under tableting pressure 6 kN using a rotary tableting machine (Aquarius 36K, Kikusui Seisakusho) to obtain core tablets. The resulting core tablets are subjected to coating by 5 mg per core tablet with aqueous suspension (concentration of solid ingredients: 10%) prepared by dissolving in purified water Opadry red 03F45055 (Nippon Colorcon) which is a premixture of Hypromellose 2910, Macrogol 6000, titanium dioxide and red ferric oxide, using a coating machine (HCF-100F, manufactured by Freund Industrial Co.) to obtain 195000 film-coated tablets containing 20 mg of Compound A per tablet and having the following prescription.

Film-coated Tablet Prescription (composition per tablet):

| Composition | Compounded Amount (mg) |
| --- | --- |
| (1) Compound A | 20.0 |
| (2) lactose | 63.0 |
| (3) crystalline cellulose | 27.0 |
| (4) carboxymethylstarch sodium | 6.0 |
| (5) hydroxypropylcellulose | 2.4 |
| (6) light silicic acid anhydride | 0.4 |
| (7) magnesium stearate | 1.2 |
| (8) Hypromellose 2910 | 3.67 |
| (9) Macrogol 6000 | 0.83 |
| (10) titanium dioxide | 0.42 |
| (11) red ferric oxide | 0.08 |
| Total | 125.0 |

[Reference Example 7]

Compound A (7875g), mannitol (11080g) and crystalline cellulose (2340g) are charged in a fluidized-bed granulator (FD-S2, Powrex Corp.), and granulated with spraying 11700g of 6% aqueous solution of hydroxypropylcellulose (HPC-L) to obtain granules. The resulting granules are charged in a granulator (powder mill P-3, Showakagaku Kikai) to obtain milled granules. To two batches (38460g) of the obtained milled granules, cross carmelose sodium (2046g) and magnesium stearate (409.2g) are added, and mixed with a blender (tumbler blender, Showakagaku Kikai).

The resulting blended granule is compressed into tablets each weighing 120 mg with 6.5 mm diameter punch under tableting pressure 6 kN using a rotary tableting machine (Aquarius 36K, Kikusui Seisakusho) to obtain core tablets. The resulting core tablets are subjected to coating by 5 mg per core tablet with aqueous suspension (concentration of solid ingredients: 10%) prepared by dissolving in purified water Opadry red 03F45055 (Nippon Colorcon) which is a premixture of Hypromellose 2910, Macrogol 6000, titanium dioxide and red ferric oxide, using a coating machine (HCF-100F, manufactured by Freund Industrial Co.) to obtain 301500 film-coated tablets containing 40 mg of Compound A per tablet and having the following prescription.

Film-coated Tablet Prescription (composition per tablet):

| Composition | Compounded Amount (mg) |
|---|---|
| (1) Compound A | 40.0 |
| (2) D-mannitol | 57.2 |
| (3) crystalline cellulose | 12.0 |
| (4) cross carmelose sodium | 6.0 |
| (5) hydroxypropylcellulose | 3.6 |
| (6) magnesium stearate | 1.2 |
| (7) Hypromellose 2910 | 3.67 |
| (8) Macrogol 6000 | 0.83 |
| (9) titanium dioxide | 0.42 |
| (10) red ferric oxide | 0.08 |
| Total | 125.0 |

[Reference Example 8]

Compound A (7875g), lactose (11080g) and corn starch (2340g) are charged in a fluidized-bed granulator (FD-S2, Powrex Corp.), and granulated with spraying 11700g of 6% aqueous solution of Hypromellose 2910 (TC-5) to obtain granules. The resulting granules are charged in a granulator (powder mill P-3, Showakagaku Kikai) to obtain milled granules. To two batches (38460g) of the obtained milled granules, crosspovidon (2046g) and magnesium stearate (409.2g) are added, and mixed with a blender (tumbler blender, Showakagaku Kikai). The resulting blended granule is compressed into tablets each weighing 120 mg with 6.5 mm diameter punch under tableting pressure 6 kN using a rotary tableting machine (Aquarius 36K, Kikusui Seisakusho) to obtain core tablets. The resulting core tablets are subjected to coating by 5 mg per core tablet with aqueous suspension (concentration of solid ingredients: 10%) prepared by dissolving in purified water Opadry red 03F45055 (Nippon Colorcon) which is a premixture of Hypromellose 2910, Macrogol 6000, titanium dioxide and red ferric oxide, using a coating machine (HCF-100F, manufactured by Freund Industrial Co.) to obtain 301500 film-coated tablets containing 40 mg of Compound A per tablet and having the following prescription.

Film-coated Tablet Prescription (composition per tablet):

| Composition | Compounded Amount (mg) |
|---|---|
| (1) Compound A | 40.0 |
| (2) lactose | 57.2 |
| (3) corn starch | 12.0 |
| (4) crosspovidon | 6.0 |
| (5) Hypromellose 2910 | 3.6 |
| (6) magnesium stearate | 1.2 |
| (7) Hypromellose 2910 | 3.67 |
| (8) Macrogol 6000 | 0.83 |
| (9) titanium dioxide | 0.42 |
| (10) red ferric oxide | 0.08 |

(continued)

| Composition | Compounded Amount (mg) |
|---|---|
| Total | 125.0 |

[Reference Example 9]

Compound A (7875g), lactose (9480g) and crystalline cellulose (4095g) are charged in a fluidized-bed granulator (FD-S2, Powrex Corp.), and granulated with spraying 11700g of 4% aqueous solution of hydroxypropylcellulose (HPC-L) to obtain granules. The resulting granules are charged in a granulator (powder mill P-3, Showakagaku Kikai) to obtain milled granules. To two batches (38330g) of the obtained milled granules, carboxymethylstarch sodium (2046g), light silicic acid anhydride (136.4g) and magnesium stearate (409.2g) are added, and mixed with a blender (tumbler blender, Showakagaku Kikai). The resulting blended granule is compressed into tablets each weighing 120 mg with 6.5 mm diameter punch under tableting pressure 6 kN using a rotary tableting machine (Aquarius 36K, Kikusui Seisakusho) to obtain core tablets. The resulting core tablets are subjected to coating by 5 mg per core tablet with aqueous suspension (concentration of solid ingredients: 10%) prepared by dissolving in purified water Opadry red 03F45055 (Nippon Colorcon) which is a premixture of Hypromellose 2910, Macrogol 6000, titanium dioxide and red ferric oxide, using a coating machine (HCF-100F, manufactured by Freund Industrial Co.) to obtain 301500 film-coated tablets containing 40 mg of Compound A per tablet and having the following prescription.

Film-coated Tablet Prescription (composition per tablet):

| Composition | Compounded Amount (mg) |
|---|---|
| (1) Compound A | 40.0 |
| (2) lactose | 49.0 |
| (3) crystalline cellulose | 21.0 |
| (4) carboxymethylstarch sodium | 6.0 |
| (5) hydroxypropylcellulose | 2.4 |
| (6) light silicic acid anhydride | 0.4 |
| (7) magnesium stearate | 1.2 |
| (8) Hypromellose 2910 | 3.67 |
| (9) Macrogol 6000 | 0.83 |
| (10) titanium dioxide | 0.42 |
| (11) red ferric oxide | 0.08 |
| Total | 125.0 |

[Experiment 1]

The sustained-release preparation of the present invention was evaluated by dissolution test.

The dissolution test was carried out for the tablets obtained in Example 1 (paddle method, 900 mL of the second disintegration solution containing 0.1% cetyltrimethylammonium bromide (CTAB) of the Japanese Pharmacopoeia (hereinafter, abbreviated as second solution containing CTAB of Japanese Pharmacopoeia), rotation speed 100 rpm). The results of the dissolution test are shown in Table 2.

[Table 2]

| Time (hr) | Dissolution Rate (%) |
|---|---|
| 0 | 0 |
| 2 | 8 |
| 4 | 17 |
| 6 | 25 |
| 8 | 33 |
| 10 | 42 |
| 12 | 50 |

(continued)

| Time (hr) | Dissolution Rate (%) |
|---|---|
| 14 | 58 |
| 16 | 65 |
| 18 | 72 |
| 20 | 78 |
| 22 | 82 |
| 24 | 90 |

[Experiment 2]

The sustained-release preparation of the present invention was evaluated by dissolution test.

The dissolution test was carried out for the film-coated tablets obtained in Examples 2 to 4 (paddle method, 900 mL of the second solution containing CTAB of Japanese Pharmacopoeia, rotation speed 100 rpm). The results of the dissolution test are shown in Table 3.

[Table 3]

| | Dissolution Rate (%) | | |
|---|---|---|---|
| Time (hr) | Example 2 | Example 3 | Example 4 |
| 0 | 0 | 0 | 0 |
| 1 | 12 | 5 | 2 |
| 2 | 29 | 14 | 5 |
| 4 | 59 | 32 | 14 |
| 6 | 82 | 50 | 24 |
| 8 | 95 | 64 | 34 |
| 10 | 100 | 75 | 44 |
| 12 | - | 83 | 53 |
| 16 | - | 92 | 70 |
| 20 | - | 94 | 83 |
| 24 | - | - | 93 |

From the above, it was shown that the sustained-release preparation of the present invention has an excellent sustained-release property. In addition, it became evident that the sustained release of the preparation can be adjusted by changing the compounding ratio of hydrophilic polymer contained in the sustained-release preparation of the present invention.

[Experiment 3]

FXa inhibitory activity of the preparation of the present invention is evaluated with cynomolgus monkey.

As control, a suspension containing Compound A prepared by the following method is used.

Compound A (375 mg) and methylcellulose (625 mg) were mixed well with a mortar, and water for injection (125 mL) was gradually added thereto to prepare a suspension.

The suspension containing Compound A prepared by the above method and two tablets obtained in Example 1 were administered orally to cynomolgus monkeys fasted overnight. In addition, before administration and at a given time course after administration, blood was collected in a syringe preloaded with sodium citrate solution from the femoral vein, and the concentration of Compound A in the obtained plasma and FXa inhibitory activity were measured.

The results obtained are shown in Table 4 and Table 5. The value of FXa inhibitory activity in the Tables represents mean value ± standard deviation (Table 4: n=3, Table 5: n=6).

[Table 4]

Administration of suspension (3 mg per 1 kg body weight)

| Time (hr) | plasma level of Compound A (nmol/mL) | FXa inhibitory activity (%) |
|---|---|---|
| 0 | 0 ± 0 | 8.00 ± 3.1 |

(continued)

Administration of suspension (3 mg per 1 kg body weight)

| Time (hr) | plasma level of Compound A (nmol/mL) | FXa inhibitory activity (%) |
|---|---|---|
| 0.5 | 0.92 ± 0.14 | 92.4 ± 0.6 |
| 1 | 0.82 ± 0.30 | 91.6 ± 2.0 |
| 2 | 0.52 ± 0.16 | 89.1 ± 3.4 |
| 4 | 0.09 ± 0.01 | 57.3 ± 5.1 |
| 8 | 0.02 ± 0.004 | 22.2 ± 0.3 |
| 24 | 0.0004 ± 0.0004 | 12.4 ± 1.7 |

[Table 5]

Sustained-release preparation (20mg administration as Compound A per cynomolgus monkey)

| Time (hr) | plasma level of Compound A (nmol/mL) | FXa inhibitory activity (%) |
|---|---|---|
| 0 | 0 ± 0 | 2.3 ± 1.1 |
| 1 | 0.04 ± 0.03 | 15.6 ± 5.4 |
| 2 | 0.12 ± 0.06 | 48.8 ± 9.3 |
| 4 | 0.13 ± 0.05 | 58.2 ± 5.3 |
| 8 | 0.10 ± 0.04 | 50.1 ± 6.7 |
| 12 | 0.07 ± 0.04 | 35.3 ± 6.2 |
| 16 | 0.15 ± 0.11 | 52.3 ± 11.8 |
| 24 | 0.03 ± 0.03 | 15.3 ± 4.8 |

From the above, it was shown that the sustained-release preparation of the present invention has an excellent sustained FXa inhibitory activity.

[Experiment 4]

The controlled-release granules obtained in Example 8 were filled in a capsule with an amount of 10 mg as Compound A per cynomolgus monkey, and administered orally to cynomolgus monkeys fasted overnight. In addition, before administration and at a given time course after administration, blood was collected in a syringe preloaded with sodium citrate solution from the femoral vein, and the concentration of Compound A in the obtained plasma and FXa inhibitory activity were measured.

The results obtained are shown in Table 6. The value of FXa inhibitory activity in the Table represents mean value ± standard deviation (n=3).

[Table 6]

| Time (hr) | plasma level of Compound A (nmol/mL) | FXa inhibitory activity (%) |
|---|---|---|
| 0 | 0 ± 0 | -3.1 ± 0.3 |
| 1 | 0.001 ± 0.0009 | -2.5 ± 0.4 |
| 2 | 0.01 ± 0.01 | 2.9 ± 1.6 |
| 4 | 0.19 ± 0.09 | 68.4 ± 10.7 |
| 8 | 0.10 ± 0.03 | 48.4 ± 6.4 |
| 12 | 0.04 ± 0.03 | 18.0 ± 6.0 |
| 24 | 0.01 ± 0.01 | 4.4 ± 3.4 |

Industrial Applicability

The sustained-release preparation, pharmaceutical composition and controlled-release preparation containing a FXa inhibitor of the present invention can inhibit appropriately FXa activity over a long period. Therefore, in that the sustained-release preparation has less side effects and an administration of once a day will suffice, it is useful as a medicine (for example, a prophylactic or therapeutic agent for thrombosis) excellent in convenience, compliance and safety.

In addition, it becomes possible to decrease formulation obstacles in the compression formulation by the process for production of the sustained-release preparation of the present invention, thus a sustained-release preparation having a good productivity can be provided.

## Claims

1. A sustained-release preparation comprising a blood coagulation factor Xa inhibitor and a hydrophilic polymer.

2. The sustained-release preparation according to claim 1, wherein the blood coagulation factor Xa inhibitor is 1-(1-{(2S)-3-[(6-chloronaphthalen-2-yl)sulfonyl]-2-hydroxypropanoyl}piperidin-4-yl)tetrahydropyrimidin-2(1H)-one.

3. The sustained-release preparation according to claim 1, wherein a content of the hydrophilic polymer in the preparation is 5 to 90% by weight.

4. A pharmaceutical composition which comprises a combination of preparations containing two or more blood coagulation factor Xa inhibitors, which have a different release rate of the blood coagulation factor Xa inhibitor.

5. The pharmaceutical composition according to claim 4, wherein the blood coagulation factor Xa inhibitor is 1-(1-{(2S)-3-[(6-chloronaphthalen-2-yl)sulfonyl]-2-hydroxypropanoyl}piperidin-4-yl)tetrahydropyrimidin-2(1H)-one.

6. The pharmaceutical composition according to claim 4, which comprises a combination of a sustained-release preparation containing a blood coagulation factor Xa inhibitor and a quick-release preparation containing a blood coagulation factor Xa inhibitor.

7. A controlled-release preparation containing a blood coagulation factor Xa inhibitor, which can maintain a plasma level of the blood coagulation factor Xa inhibitor at 1.25 nmol/mL or less over one hour to 24 hours after the administration and can reduce the blood coagulation factor Xa activity by 10% or more than usual over one hour to 24 hours after the administration.

8. The preparation according to claim 7, wherein the blood coagulation factor Xa inhibitor is 1-(1-{(2S)-3-[(6-chloronaphthalen-2-yl)sulfonyl]-2-hydroxypropanoyl}piperidin-4-yl)tetrahydropyrimidin-2(1H)-one.

9. The preparation according to claims 1 or 7, which is for prevention or treatment of thrombosis.

10. The preparation according to claims 1 or 7, which is a secondary prophylactic agent of acute coronary syndrome.

11. A process for production of a sustained-release preparation, comprising:

    a) a step for granulating a mixture comprising an active ingredient and mannitol with spraying an aqueous solution of hydroxypropyl cellulose, and
    b) a step for formulating a mixture comprising the granulated material obtained in step a) and 2 or more hydrophilic polymers to obtain a formulated substance.

12. The process for production according to claim 11, wherein the 2 or more hydrophilic polymers are hydroxypropyl cellulose and hydroxypropyl methylcellulose.

13. The process for production according to claim 11, wherein the formulation in step b) is carried out by a compression formation and formation pressure in the compression formation is 3 to 14kN.

14. The sustained-release preparation obtained by the process for production according to claim 13, wherein the absolute hardness of the formulated substance obtained in step b) is 0.8 to 4.5N/mm$^2$.

15. A controlled-release preparation having a controlled-release coating layer on a formulated substance containing a blood coagulation factor Xa inhibitor.

16. The controlled-release preparation according to claim 15, wherein the blood coagulation factor Xa inhibitor is 1-(1-{(2S)-3-[(6-chloronaphthalen-2-yl)sulfonyl]-2-hydroxypropanoyl}piperidin-4-yl)tetrahydropyrimidin-2(1H)-one.

**17.** The controlled-release preparation according to claim 15, wherein the controlled-release coating layer is a controlled-release coating layer containing a pH-dependently soluble polymer.

**INTERNATIONAL SEARCH REPORT**

International application No.
PCT/JP2007/073031

A. CLASSIFICATION OF SUBJECT MATTER
*A61K9/22*(2006.01)i, *A61K31/506*(2006.01)i, *A61K47/10*(2006.01)i, *A61K47/38*(2006.01)i, *A61P7/02*(2006.01)i

According to International Patent Classification (IPC) or to both national classification and IPC

B. FIELDS SEARCHED

Minimum documentation searched (classification system followed by classification symbols)
A61K9/22, A61K31/506, A61K47/10, A61K47/38, A61P7/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | WO 98/003202 A1 (Daiichi Pharmaceutical Co., Ltd.),<br>29 January, 1998 (29.01.98),<br>Example 4<br>& EP 966973 A1 | 1,3,7,9,10,15<br><br>1-4,6 |
| X<br>Y | WO 2000/043041 A1 (Yamanouchi Pharmaceutical Co., Ltd.),<br>27 July, 2000 (27.07.00),<br>Comparative example 2; page 2, lines 17 to 20<br>& AU 200030747 A | 1,7,9,10<br>1-4,6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>29 January, 2008 (29.01.08) | Date of mailing of the international search report<br>12 February, 2008 (12.02.08) |
| Name and mailing address of the ISA/<br>Japanese Patent Office<br><br>Facsimile No. | Authorized officer<br><br><br>Telephone No. |

Form PCT/ISA/210 (second sheet) (April 2007)

**INTERNATIONAL SEARCH REPORT**

International application No.
PCT/JP2007/073031

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | WO 96/016940 A1 (Yamanouchi Pharmaceutical Co., Ltd.),<br>06 June, 1996 (06.06.96),<br>Full text; particularly, page 29, lines 9 to 11; page 30, lines 2 to 8<br>& EP 798295 A1 & US 5869501 A | 1,3,7,9,10,<br>15,17<br>1-4,6 |
| X<br><br>Y | WO 2004/048363 A1 (Takeda Chemical Industries, Ltd.),<br>10 June, 2004 (10.06.04),<br>Full text; particularly, page 53, line 9 to page 54, line 25<br>& JP 2004-182730 A & EP 1564213 A1<br>& US 2007/0004736 A1 | 1-3,7-10,<br>15-17<br>1-6 |
| X<br><br>Y | WO 2005/113504 A1 (TAKEDA PHARM CO., LTD.),<br>01 December, 2005 (01.12.05),<br>Full text; particularly, page 69, line 25 to page 71, line 18<br>& EP 1748985 A1 | 1-3,7-10,<br>15-17<br>1-6 |
| Y | JP 11-021233 A (Taisho Pharmaceutical Co., Ltd.),<br>26 January, 1999 (26.01.99),<br>Claims<br>(Family: none) | 4-6 |
| Y | Hiroaki NAKAUE, "Cellulose-kei Hokaizai no Ryushi Sekkei to Johojo eno Oyo, Ryushi Sekkei to Seizai Gijutsu", Kabushiki Kaisha Yakugyo Jihosha, 30 October, 1993 (30.10.93), pages 63 to 68 | 1-3 |
| X | JP 2000-336026 A (Lion Corp.),<br>05 December, 2000 (05.12.00),<br>Example 4<br>(Family: none) | 11-14 |
| A | JP 04-159217 A (Freund Industrial Co., Ltd.),<br>02 June, 1992 (02.06.92),<br>Full text<br>& EP 482576 A & US 5480654 A<br>& JP 3011752 B2 | 11-14 |
| A | JP 05-092918 A (Takeda Chemical Industries, Ltd.),<br>16 April, 1993 (16.04.93),<br>Full text<br>& JP 2820829 B2 | 11-14 |

Form PCT/ISA/210 (continuation of second sheet) (April 2007)

**INTERNATIONAL SEARCH REPORT**

International application No.
PCT/JP2007/073031

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The inventions of claims 1-3, the inventions of claims 4-6, the inventions of claims 7 and 8, and the inventions of claims 15-17 relate to sustained release preparations each comprising a blood coagulation factor Xa inhibitor, which can release the inhibitor by different means. The inventions of claims 11-14 relate to a method for producing a sustained release preparation comprising an active ingredient.

A preparation comprising a blood coagulation factor Xa inhibitor which contains a hydrophilic polymer and appears to be a sustained release preparation is disclosed in Example 4 in Document 1 and Comparative Example 2 in Document

(continued to extra sheet)

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

INTERNATIONAL SEARCH REPORT

International application No.
PCT/JP2007/073031

Continuation of Box No.III of continuation of first sheet(2)

2. Taking this fact into consideration, it cannot be considered that the above-stated groups of inventions shear any common technical feature which defines a contribution that makes over the prior art.

Therefore, it cannot be considered that these groups of inventions are so related as to form a single general inventive concept.

Document 1: WO 98/003202 A1
Document 2: WO 2000/043041 A1

<Subject of search>

Claims 1, 3, 4, 6, 7, 9, 10, 15 and 17 relate to a reparation comprising a compound which is defined by a desired property "a blood coagulation factor Xa inhibitor", and the "blood coagulation factor Xa inhibitor" includes within its scope all of the compounds having the property. However, among these compounds, those which are supported by the description in the meaning within PCT Article 6 and disclosed in the meaning within PCT Article 5 appear to be limited to an extremely small part of the claimed compounds.

Further, even though the common technical knowledge at the time of filing the present application is taken into the consideration, it appears that the scope of the compound having the property "a blood coagulation factor Xa inhibitor" cannot be specified. Thus, claims 1, 3, 4, 6, 7, 9, 10, 15 and 17 do not comply with the requirement of clearness under PCT Article 6, too.

Claims 7-10 relate to "a sustained release preparation comprising a blood coagulation factor Xa inhibitor" which has a specific pharmacokinetics, and the sustained release preparation includes within its scope all of the preparations having the pharmacokinetics. However, among these preparations, those which are supported by the description in the meaning within PCT Article 6 and disclosed in the meaning within PCT Article 5 appear to be limited to an extremely small part of the claimed preparations.

Further, even though the common technical knowledge at the time of filing the present application is taken into the consideration, it is impossible to specify the scope pf the sustained release preparation. Therefore, claims 7-10 do not comply with the requirement of clearness under PCT Article 6, too.

Such being the case, the search was made mainly on the parts specifically described in and disclosed in the description, namely a sustained release preparation comprising a compound represented by the formula (1) described in Paragraph [0006] of the present description or a compound A described in the working example of the present application (i.e., a compound disclosed in Example 11 in WO 96/16940) and a hydrophilic polymer. With regard to claims 2, 5, 8, 11-14 and 16, the search was made completely.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9616940 A


### Non-patent literature cited in the description


- Medicine Ranking. Mikusu Co, 2002